# EUROPÄISCHE PATENTSCHRIFT

(11) **EP 1 414 335 B1**
(45) Veröffentlichungstag und Bekanntmachung des Hinweises auf die Patenterteilung: **14.09.2011**
(21) Anmeldenummer: 02754427.9
(22) Anmeldetag: 24.07.2002
(51) Int. Cl.: A61B 5/00, A61B 5/0404, A61B 5/044, A61B 5/05

(54) **VORRICHTUNG ZUR UNTERSUCHUNG UND ÜBERWACHUNG VON VITALPARAMETERN DES KÖRPERS**
DEVICE FOR VERIFYING AND MONITORING VITAL PARAMETERS OF THE BODY
DISPOSITIF POUR CONTROLER ET SURVEILLER DES PARAMETRES VITAUX DU CORPS

(30) Priorität: 26.07.2001 DE 10136355
(43) Veröffentlichungstag der Anmeldung: 06.05.2004
(73) Patentinhaber: Rahe-Meyer, Niels, 30177 Hannover (DE)
(72) Erfinder: Rahe-Meyer, Niels, 30177 Hannover (DE)
(74) Vertreter: Chambosse, Hans-Joachim
(86) Internationale Anmeldenummer: PCT/DE2002/002718
(87) Internationale Veröffentlichungsnummer: WO 2003/011124

(56) Entgegenhaltungen:
- EP-A- 1 031 317
- WO-A-88/05282
- WO-A-89/00024
- WO-A-96/14014
- US-A- 4 270 547
- US-A- 4 974 599
- US-A- 5 882 303
- US-A- 6 167 290

## Beschreibung

Die Erfindung betrifft eine Vorrichtung zur Untersuchung und Überwachung von Vitalparametern des menschlichen oder tierischen Körpers in Form eines kompakten, vorn Benutzer einhändig faßbaren und einsetzbaren Diagnosegeräts ohne die Notwendigkeit von Kabeln und/oder Schläuchen.

Bei bekannten Untersuchungs- und Überwachungsgeräten sind an dem zu untersuchenden Körper mehrere Elektroden angebracht, die elektrische Spannungsschwankungen des Körpers aufnehmen. Üblicherweise wird dabei zur Erhöhung der Anzahl der Meßvektoren gleichsinnig die Anzahl der Elektroden erhöht. Diese Elektroden sind bei den meisten der bekannten Geräte mittels Kabeln an Verstärkereinheiten für eine Verstärkung der Signale und an getrennten Auswertegeräten angeschlossen. Diese bekannten Untersuchungs- und Überwachungsgeräte sind insbesondere durch die erwähnten Kabelverbindungen und die getrennten Verstärkereinheiten und Auswertegeräte, zudem durch die räumlichen Abmessungen dieser Bauteile mit erheblichen Nachteilen verbunden. Zum einen behindern die Kabel die untersuchte Person. Vor allem aber wird der Einsatz durch den Arzt behindert, da die Geräte jeweils ortsgebunden bei der einzelnen untersuchten Person sind und nicht vom Arzt ständig mitgeführt und in kürzester Zeit ohne sonstige Anschlüsse an einer Vielzahl von Patienten eingesetzt werden können.

Aus der DE 43 29 898 A1 ist ein kabelloses Diagnose- und Überwachungsgerät bekannt. Bei diesem kann auf eine elektrische Kabelverbindung der Elektroden zur Auswertestation verzichtet werden, indem die am untersuchten Körper angebrachten Elektroden mit Sendeeinheiten und Antennen ausgerüstet sind, durch die sie mit der getrennten Auswertestation drahtlos in Verbindung stehen und aufgenommene Signale zur Auswertestation senden. Ebenso verfügt die Auswertestation über Empfangseinheiten und Antennen. Aus dieser Druckschrift ist auch bekannt, in einem Elektrodengehäuse mehrere Elektrodenpins zur Aufnahme elektrischer Potentialschwankungen an verschiedenen Stellen mit verschiedenen Vektoren anzubringen. Dieses Diagnose- und Überwachungsgerät hat gleichwohl noch erhebliche Nachteile. Zum einen ist es technisch kompliziert und teuer durch die erforderlichen Sender, Empfänger, Detektoren, Codier- und Decodiereinheiten u. a.. Die, auch bauartbedingt relativ großen Elektroden müssen am Körper dauerhaft, wenn auch lösbar befestigt werden, wobei für die Untersuchung verschiedener Körperpartien jeweils getrennte Elektroden mit den erforderlichen Sendern und Empfängern benötigt werden. Ein weiterer maßgeblicher Nachteil ist die räumlich von den Elektroden getrennte und ortsfeste Anordnung der relativ großen Auswertestation, zumal diese die Display-, Speicher- und Alarmeinheiten beinhaltet. Sie ist deshalb für die Untersuchungsperson, also insbesondere den Arzt, nicht jederzeit zugänglich und beobachtbar, weil sie nicht von ihr mitgeführt werden kann. Und schließlich ist von großem Nachteil, daß nur wenige Untersuchungsfunktionen (Sensorarten) in die Vorrichtung integriert sind, insbesondere keine Auskultation, keine Perkussion, keine Messung der Sauerstoffsättigung des Blutes und der Rekapillarisierungszeit möglich sind.

Bekannt ist schließlich auch aus der DE 40 12 874 C2 eine am Körper tragbare Blutdruckmeßvorrichtung mit einer Langzeit-EKG-Meßeinrichtung. In dieser sind der zur automatischen Blutdruckmessung erforderliche Geräteteil einschließlich Manschette und Pumpe sowie die Auswerte- und Steuerschaltung in einem System zusammengefaßt. Diese Vorrichtung ist speziell für eine automatische Langzeit-EKG- und Blutdruckmessung ausgelegt und hat vorrangig die Aufgabe, Blutdruckmessungen im Zusammenhang mit EKG.Veränderungen vorzunehmen. Schon wegen der erforderlichen Manschette und der zu ihrer Bedienung dienenden Pumpe muß das Gerät jeweils dem Einzelpatienten zugeordnet und von ihm getragen werden. Außerdem sind bei dieser Vorrichtung die Elektroden und Sensoren der Langzeit-EKG-Meßeinrichtung mittels Kabeln mit der Langzeit-EKG-Meßeinrichtung verbunden.

Eine Vorrichtung gemäß der Präambel des Anspruchs 1 ist aus den Dokumenten WO-A-96/14 014, WO-A-88/05 282 und EP-A-1 031 317 bekannt.

Aufgabe der Erfindung ist es, unter Vermeidung der Nachteile bekannter Diagnose- und Überwachungsgeräte eine Vorrichtung zur Schnelldiagnose der wichtigsten Vitalparameter des menschlichen oder tierischen Körpers, insbesondere zur Untersuchung von Herz und/oder Lunge in Ausdehnung, Beschaffenheit und Funktion, sowie zur Dauerüberwachung des Untersuchten in einer solchen kompakten Form und ohne Kabel- und/oder Schlauchverbindungen zu schaffen, daß es von dem Anwender, insbesondere dem Arzt, ständig mitgeführt werden und sofort ohne sonstige Anschlüsse eingesetzt werden kann, sowie die Messung von Oxygenierungswerten und Kreislaufwerten des menschlichen oder tierischen Körpers unter Durchleuchtung und Ausübung von Druck auf einen Körperabschnitt mit anschließender Druckentlastung.

Diese Aufgabe wird durch die erfindungsgemäße Vorrichtung gemäß Patentanspruch 1 gelöst. Sonstige zweckmäßige Ausgestaltungen werden gemäß den weiteren Patentansprüchen erreicht.

Nachfolgend wird die Erfindung anhand von Zeichnungen erläutert, wobei lediglich in den Figuren 13-16 Ausführungsformen der erfindungsgemäßen Vorrichtung dargestellt werden. Es zeigen :
- Fig. 1:: eine nicht erfindungsgemäβe Vorrichtung zur Untersuchung und Überwachung von Vitalparame- tern im Längsschnitt;
- Fig. 2:: die Diagnosevorrichtung gemäß Fig. 1 von der auf das Untersuchungs- objekt aufzulegenden Unterseite her gesehen;
- Fig. 3:: die Diagnosevorrichtung gemäß Fig. 1 in Aufsicht von oben mit aufge- klapptem Bildschirm sowie Bedien- und Anzeigeteil;
- Fig. 4:: die Diagnosevorrichtung in abgeänderter Ausführungsform mit fest inte- griertem Bildschirm und klappbarem Schutzdeckel sowie einer nicht er- findungsgemäßen ausziehbaren Sensorelektrode;
- Fig. 5:: die Diagnosevorrichtung gemäß Fig. 4 in der Ansicht von unten, wie in Fig. 2;
- Fig. 6:: eine weitere nicht erfindungsgemäße Diagnosevorrichtung in Seitenansicht im Längsschnitt, wie in Fig. 1, jedoch mit Schallzylinder und Schnittstel- le am Diagnosegerät und fest integriertem Bildschirm sowie mit ange- setzten externen Sensorelektroden;
- Fig. 7:: die Diagnosevorrichtung gemäß Fig. 6 in der Aufsicht von oben mit An- zeige und Bedienfeld sowie Bildschirm;
- Fig. 8:: die Diagnosevorrichtung gemäß Fig. 6 in der Ansicht von unten, wie in Fig. 2;
- Fig. 9:: eine weitere nicht erfindungsgemäße Diagnosevorrichtung mit auswech- selbarem Schalltrichter und auswechselbarem Schalltrichter und aus- wechselbarer Membran für die akustische Signalaufnahme;
- Fig. 10:: als Detail von Fig. 9 einen Schalltrichter sowie eine Membran vor dem Einsatz;
- Fig. 11:: als Detail zu Fig. 9 einen anderen Schalltrichter ohne Membran;
- Fig. 12:: ein nicht erfindungsgemäßes, seitlich an die Diagnosevorrichtung an- setzbares Perkussionsmodul;
- Eig. 13:: die erfindungsgemäße Diagnosevorrichtung als Längsschnitt mit inte- griertem Perkussionselement mit elektromagnetisch angetriebenern Schlägel sowie auf der Oberseite über dem Bedien- und Anzeige- feld/Bildschirm aufgesetzter Aufnahmeeinheit mit Andruckvorrichtung für einen eingoschobenen Fingerabschnitt und Lichtsonder/-empfanger;

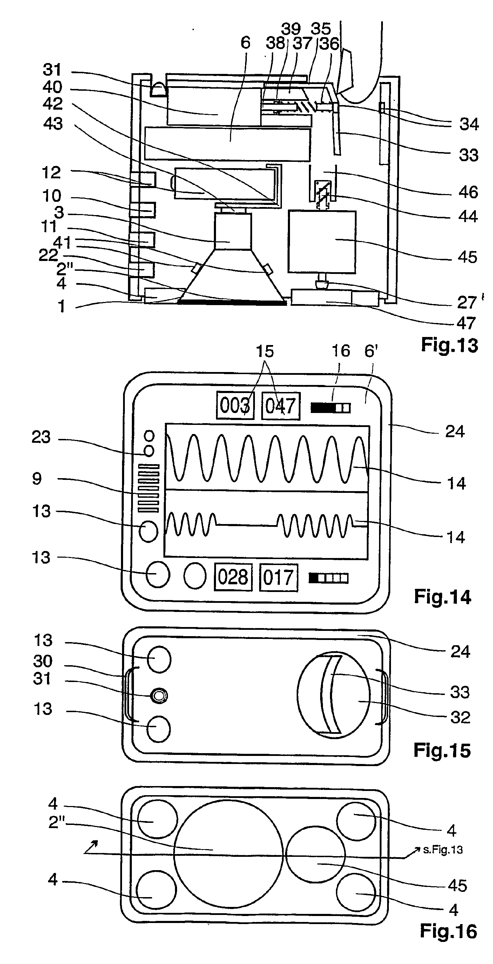

- Fig. 14:: das Bedien- und Anzeigefeld mit Bildschirm der Diagnosevorrichtung gemäß Fig. 13 in Aufsicht von oben nach Abnahme der Andruckvorrich- tung und Aufnahmeeinheit;
- Fig.15:: die Diagnosevorrichtung gemäß Fig. 13 in Aufsicht von oben auf die Andruckvorrichtung;
- Fig. 16:: die Diagnosevorrichtung gemäß Fig. 13 in Aufsicht von unten mit Meß- elektroden und Schalltrichter.

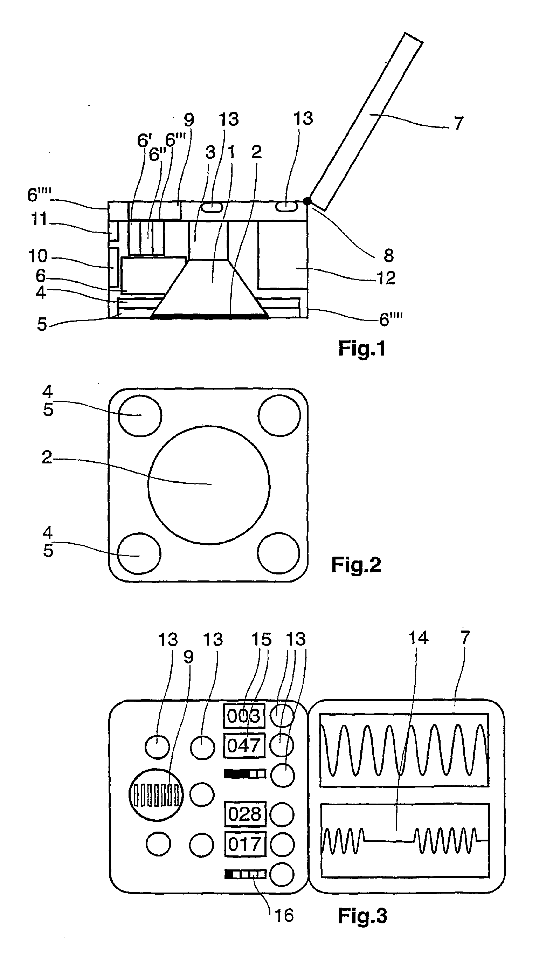

Bei der in Fig. 1 bis 3 dargesteliten Diagnosevorrichtung sind in einem Gehäuse die Aufnahmeeinheit für akustische Signale des Körpers (1, 2, 3) sowie die Aufnahmeeinheit für elektrische Signale in Form von Spannungsschwankungen und/oder von Impedanzwerten und/oder für Temperaturwerte, bestehend aus vier Meßelektroden (4) mit Kontaktmittelraum (5), eingesetzt, wobei der Schalltrichter und die Meßelektroden mit Kontakt. mittelraum bei Benutzung der Vorrichtung dem zu untersuchenden Objekt zu gerichtet sind. Dabei liegen die Membran (2) des Schalltrichters und der Abschluß des Kontaktmittelraums (5) der Meßelektroden (4) in einer horizontalen Ebene. Auf der Oberseite der Vorrichtung befindet sich das Bedien- und Anzeigefeld mit den Bedientasten (13), dem Lautsprecher (9), Displaydigitalfeldern (15) und den Displayamplitudenbalken (16). Mittels eines Scharniers (8) ist an einem Außenrand des Gehäuses der Diagnosevorrichtung umlegbar der Bildschirm (7) angesetzt, der Displaykurvenfelder (14) aufweist und außerhalb der Benutzung um das Scharnier (8) auf das Bedien- und Anzeigefeld aufgelegt wird. Im Gehäuse der Diagnosevorrichtung sind - in den Zeichnungen nicht im einzelnen dargestellt - analoge und digitale Elektronik zur Verstärkung der Meßwerte, zur Analyse der Meßwerte und zu deren Darstellung eingesetzt. Ferner befindet sich im Gehäuse der Diagnosevorrichtung ein Element zur Datenspeicherung. Die Aufnahmeeinheit für akustische Signale besteht aus dem Schallzylinder (1) mit aufgesetzter Membran (2) und dem angesetzten Mikrophon (3). Schließlich befindet sich im Gehäuse ein Fach zur Aufnahme der Stromversorgungselemente wie Batterien oder Akkus (12). Wie schematisch in Fig. 1 dargestellt, hat die gezeigte Diagnosevorrichtung zusätzlich an einer Außenseite des Gehäuses Anschlußbuchsen bzw. Schnittstellen für den Anschluß von Ohrhörern/Kopfhörern (10) und für den Anschluß an einen Computer (11). Das Mikrophon (3) des Schalltrichters steht mit dem Lautsprecher (9) im Bedien- und Anzeigefeld in Verbindung.

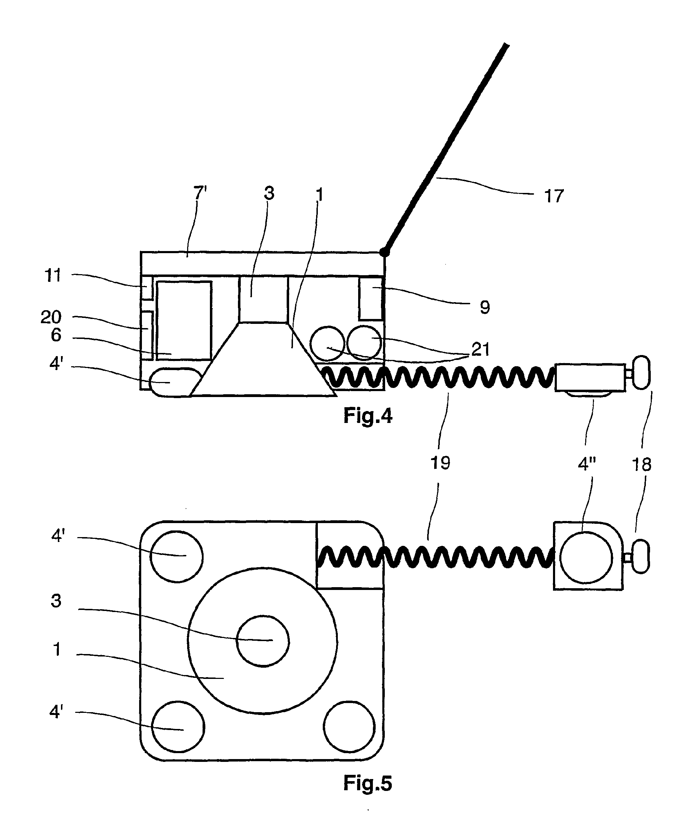

### Bei der in Fig. 4 und 5 dargestellten

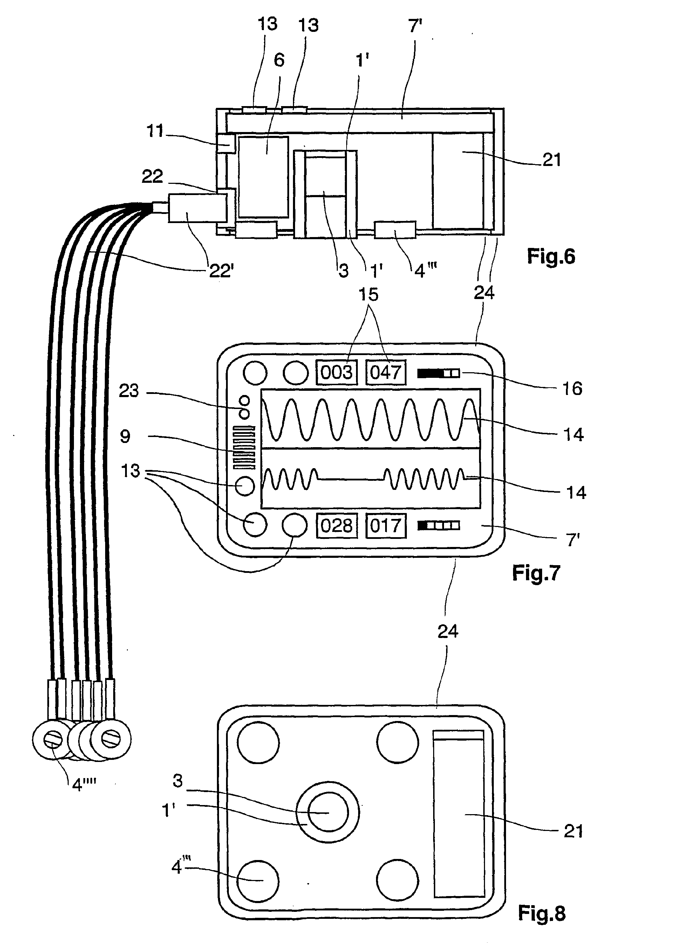

Diagnosevorrichtung ist anstelle der klappbar angesetzten Vorrichtung zur visuellen Wiedergabe der von der Aufnahmeeinheit für elektrische Signale und/oder für Temperaturwerte aufgenommenen und ausgeweiteten Signale die Vorrichtung in Form des Bildschirms (7) mit Displaykurvenfeldern (14) in das Bedien und Anzeigefeld integriert (7') und entsprechend Fig. 7 ausgestaltet. Zum Schutz des Bedien- und Anzeigefeldes nebst Bildschirm ist ein Schutzdeckel (17) klappbar an einem oberen Seitenrand der Vorrichtung angeordnet. Im übrigen kann die Diagnosevorrichtung mit der in Fig.1 bis 3 dargestellten übereinstimmen. In Fig. 4 sind jedoch in weiterer Ausgestaltung statt der im Gehäuse versenkt angeordneten Meßelektroden (4) mit Kontaktmittelraum (5) Meßelektroden (4') vorgesehen, die an der Unterseite des Gehäuses in einer Ebene mit dem Schalltrichter (1) nebst Membran (2) liegen und zweckmäßigerweise leicht gewölbt vorstehen. Ferner ist in Fig. 4 und 5 dargestellt, daß eine der Elektroden lösbar an der Unterseite des Gehäuses als Meßelektrode (4") mit Verbindungskabel (19) zur Diagnosevorrichtung eingesetzt ist und so getrennt vom übrigen Gerät an einer zu untersuchenden Stelle des Körpers allein oder unter gleichzeitigem Einsatz des Gerätes mit den weiteren Meßelektroden an einer anderen Körperstelle angesetzt werden kann. Zur Erleichterung der Bedienung der Meßelektrode (4") hat diese einen Handgriff (18). Bei der hier gezeigten Ausführungsform ist außerdem der Lautsprecher (9) an anderer Stelle des Gehäuses der Diagnosevorrichtung angeordnet und statt des Akkufachs (12) der Batterieraum (21) vorgesehen, Schließlich ist am Gehäuse zusätzlich zur Computerschnittstelle (11) oder an deren Stelle eine Schnittstelle (20) zu einem Drucker vorhanden.

Bei der in den Fig. 6 bis 8 dargsstellten Vorrichtung ist statt des Schalltrichters (1) mit Membran (2) ein Schallzylinder (1') eingesetzt, der gleichzeitig als Halterung des nachgeschalteten Mikrophons (3) dient. Ferner ist eine Schnittstelle (22) zum Anschluß externer Vorrichtungen und Geräte vorgesehen. Schließlich sind statt der Meßelektroden (4) nicht gewölbte Meßelektroden (4"') verwandt. Das Bedien- und Anzeigefeld mit integriertem Bildschirm ist auch bei dieser Ausführungsform entsprechend Fig. 7 gestaltet, kann jedoch auch gemäß Fig. 1 und 3 ausgebildet sein.

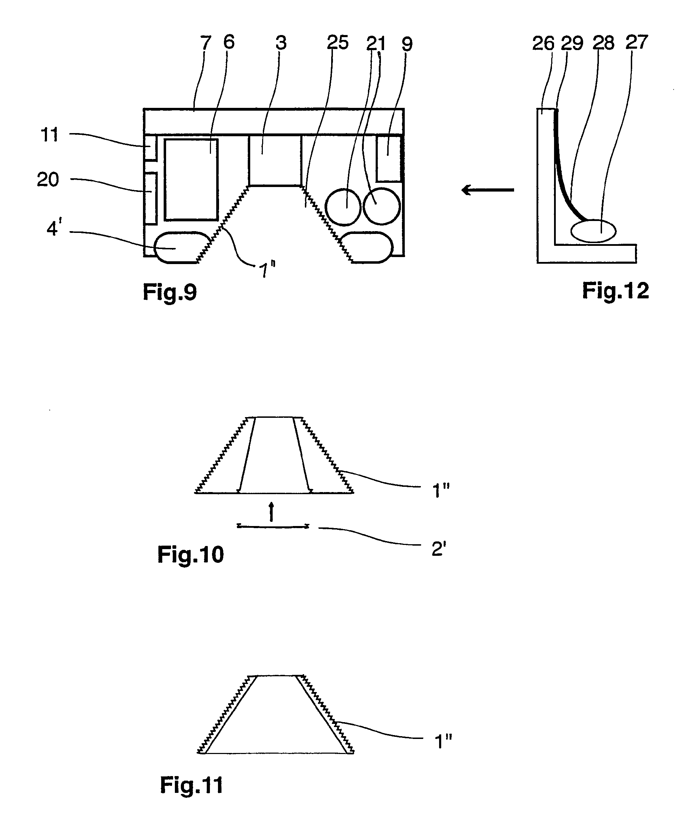

Die Fig. 9 bis 11 zeigen die Diagnosevorrichtung in einer Weiterbildung der Aufnahmeeinheit für akustische Signale die Ausstattung mit auswechselbarem Schalltrichter (1 ") und auswechselbarer Membran (2'), wobei der jeweils eingesetzte Schalltrichter mittels eines Gewindes, eines Bajonettverschlusses oder in sonstiger bekannter Weise im Gehäuse der Diagnosevorrichtung eingesetzt und mit dem Mikrophon (3) verbunden wird.

In Fig. 12 ist als zusätzliches Bauteil ein nicht erfindungsgemäßes Perkussionsmodul (26 bis 29) gezeigt, das an sämtliche Ausführungsformen der Diagnosevorrichtung anzusetzen ist. Dieses besteht aus dem Winkelprofil (26), vorzugsweise aus Metall, dessen Schenkel von rechtwinklig zueinander stehenden Flächen gebildet werden. Der senkrecht stehende Schenkel entspricht in seiner Höhe im dargestellten Ausführungsbeispiel, Jedoch nicht unabdingbar, der Höhe der Außenwände der Diegnosevorrichtung; mit der Außenfläche dieses Schenkels wird das Modul fest, jedoch lösbar, mit einer Außenwand der Diagnosevorrichtung verbunden, und zwar derart, daß der waagerechte Schenkel des Moduls In einer waagerechten Ebene mit der dem untersuchten Körperteil zugewandten Unterseite der Diagnosevorrichtung verläuft und dadurch ebenfalls auf dem Untersuchungsobjekt aufliegt. An dem senkrechten Schenkel des Profils (26) ist an dessen Innenseite im oberen Bereich (29) die Blattfeder (28) angeordnet, die an ihrem unteren Ende den Schlägel (27) hat. Dieser Schlägel liegt mit Federkraft auf der Innenseite des waagerechten Schenkels des Profils (26) auf. Durch Anheben der Feder (28) mit dem Schlägel (27) von Hand bei der Untersuchung wird beim Zurückschnellen auf den waagerechten Schenkel ein Schleggeräusch erzeugt, das vom Körper bzw. untersuchten Körperteil reflektiert und dabei von der akustischen Aufnahmevorrichtung (1, 2, 3; 1', 3; 1", 2', 3) aufgenommen und zur Verarbeitung weitergegeben wird.

In den Fig. 13 bis 16 ist die erfindungsgemäße Vorrichtung zur Untersuchung und Überwachung von Vitalparametern des menschlichen oder tierischen Körpers dargestellt. Bei dieser ist zum einen, wie aus Fig. 13 ersichtlich, die akustische Aufnahmevorrichtung (1, 2, 3) zugleich durch elaktrisch-elektronische Elemente ergänzt, indem die Membran (2") vor dem Schalltrichter (1) an ihrer dem Schalltrichter zu gerichteten Innenseite lichtreflektierend ist und am Schalltrichter (1) einander gegenüberliegende Lichtsender (41) und Lichtempfänger (41') angeordnet sind. Entsprechend der Bewegung der Membran (2") beim Ansatz an das untrsuchte Körperteil - wie zum Beispiel bei

Spannungszuständen der untersuchten Bauchdecke eines Patienten - werden die Lichtreflektion und deren unterschiedliche Werte durch die Elemente (41, 41') gemessen und zur Auswertung weitergeleitet zusätzlich zu den akustischen Signalen. Anstelle der lichtreflektierenden Membran (2") in Kombination mit Lichtsender (41) und Lichtempfänger (41') kann die Membran (2) aber auch mit einem Piezoelement oder einem Wirbelstromsensor verbunden sein, die die Bewegung bzw. Stellung der Membran bei der Untersuchung in elektrisch-elektronische Signale umsetzen. Anstelle oder zusätzlich zu diesen Vorrichtungselementen (2", 41, 41') können Oberflächenveränderungen am untersichten Körperteil auch durch Druck auf den Schalltrichter (1) gemessen werden, der verschiebbar im Gehäuse gelagert ist und den auf ihn ausgeübten Druck auf einen Druckaufnehmer (42, 43) weitergibt.

Weiterhin ist in Fig. 13 das Perkussionsmodul als Schallerzeugungsteil (27', 44 bis 47) in der Diagnosevorrichtung und deren Gehäuse integriert. Dabei wird der Schlägel (27') zur Schallerzeugung durch einen elektromagnetischen Antrieb (44, 46, 46) bewegt und schlägt zur Erzeugung des Schalls auf die an der Unterseite der Diagnosevorrichtung auf einer Ebene mit der Membran (2, 2") zur akustischen Aufnahmeeinheit liegenden Anschlagfläche (47).

Zusätzlich ist gemäß Fig. 13 zur Messung von Oxygenierungswerten und Kreislaufwerten des menschlichen oder tierischen Körpers, insbesondere der arteriellen Sauerstoffsättigung, der arteriellen kapillären und venösen Blutdruckwerte und der Rekapillarisierungszeit auf die vorstehend beschriebene Diagnosevorrichtung eine Aufnahmeeinheit für die Ermittlung der Lichtdurchgangswerte bei einem untersuchten Körperabschnitt und der Korrelierung der Lichtdurchgangswerte (31 bis 40) aufgesetzt oder über ein seitliches Scharnier klappbar angeordnet. Diese Aufnahmeeinheit hat einen Einlaß zum Einschieben eines Fingerabschnitts, wobei dieser mit seiner einen Seite an einer Außenwand des Moduls und mit der ihr gegenüberliegenden Seite des Körperteils an einer Schale (33) anliegt, die mit einer Andruckvorrichtung (36 bis 40) verbunden und mit dieser In der Führungsschiene (35) waagerecht beweglich geführt ist. Dabei wird die Schale (33) von der Andruckwelle (38) und dem Andruckmotor (40) gegen den eingesetzten Fingerabschnitt gedrückt. Die Andruckkraft wird über in Regelelement (39) an der Andruckswelle (38) geregelt, wobei das Element gleichzeitig die Höhe der Andruckskraft mißt. An der Schale (33) sind in Richtung des einzusetzenden Fingerabschnitts ein oder mehrere Lichtsender (34) angesetzt, die mit einem entsprechenden Lichtempfänger (34') auf der gegenüberliegenden Außenwand zusammenwirkt. Die von diesem Sensorenpaar (34, 34') bei der Durchleuchtung des untersuchten Körperabschnitts ermittelten und korrelierten Lichtdurchgangswerte werden als Signale an die Auswerteelektronik der Diagnosevorrichtung weitergegeben.

Die erfindungsgemäße Vorrichtung ermöglicht die Untersuchung und einen umfassenden vergleichenden Überflick über die Vitalparameter des Patienten, unter anderem mit Elektrokardiogramm, Blutdruck- bzw. Kreislaufwerte, Oxygenierungswerte (Sauerstoffsättigung des Blutes), Rakapillarisierungszeit, Tokographie und Auskultation in einem kompakten Gerät, das von der Untersuchungsperson, insbesondere vom Arzt, ständig mitgeführt werden kann. Es enthält alle zu einer solchen umfassenden Diagnose und Überwachung erforderlichen Aufnahme-, Weiterieitungs-, Auswertungs- und Speicherungsvorrichtungen für die ermittelten elektrischen und akustischen Signale des untersuchten Körpers bzw. Körperteils sowie Anzeigefelder für die ermittelten und ausgewerteten Signale. Die Speicherung und die optische und akustische Darstellung der Daten ermöglicht eine vergleichende Beurteilung der Messungen, insbesondere von EKG sowie Herztönen und -geräuschen, von Auskultation und Perkussion, und Messungen - z. B. Auskultation und Perkussion - im Seitenvergleich. Die Vorrichtung kann jederzeit bei einer Vielzahl von Patienten eingesetzt werden und ermöglicht eine sofortige Analyse mechanischer, akustischer und elektrischer Ereignisse bzw. Signalen, insbesondere die Analyse von Herz und Lunge und ihrer Funktion. Zudem ermöglicht die Speicherfunktion (Memory-Funktion) der Vorrichtung auch eine vergleichende Analyse von zwei gleichzeitigen Ereignissen oder auch zeitlich getrennter Ereignisse. Dadurch bringt die Vorrichtung erhebliche Vorteile gegenüber den bekannten Diagnose- und Überwachungsgeräten, namentlich auch in der Notfallmedizin, bei der klinischen Aufnahmeuntersuchung eines Patienten, bei Arztvisiten in der Klinik und auch in der Arztpraxis.

### BEZUGSZEICHENLISTE

1. Schalltrichter
   1'. Schallzylinder
   1". Wechselschallkopf
2. Membran
   2'. Wechselmembran
   2". Beschichtete Membran
3. Mikrophon
4. Meßelektrode (und Thermosensor)
5. Elektrodengrube für Kontaktpaste
6. Analoge und digitale Elektronik
   6'. Triggerelement
   6". Datenspeicherelement
   6"'. Filterelement
   6"". Abgeschirmtes Gehäuse
7. Bildschirm
8. Scharnier zur Aufklappen des Bildschirms
9. Lautsprecher (zur Wiedergabe von Signalen und/oder für akustische Alarmmeldung)
10. Schnittstelle zum Computer
11. Schnittstelle zum Ohrhörer beziehungsweise Kopfhörer
12. Akkumulator beziehungsweise Schnittstelle zum Netz
13. Bedientasten
   Ein/Aus
   Wahltasten von einzelner elektrischer Ableitung oder Mikrophon zum Display Wahltaste von einzelner elektrischer Ableitung oder Mikrophon zum Lautsprecher Verstärkertasten für Display und Lautsprecher Filtertasten
   Memorytasten
   Kalibiersignalauslösung
14. Displaykurvenfelder
15. Displaydigitalfelder
   Frequenz
   Amplitude
16. Displayamplitudenbalken
17. Schutzdeckel
18. Griff
19. Internes Elektrodenkabel
20. Schnittstelle zum Drucker
21. Batterienfach
22. Schnittstelle zu externen Elektrodenkabeln
   22'. Externe Elektrodenkabel
   22". Externe Elektrodenhalterung
23. LCD-Anzeigen
24. Gehäusearmierung
25. Schallkopfhalterung
26. Perkussionsrahmen
27. Perkussionsschlägel
28. Blattfeder
29. Blattfederankerpunkt
30. Gurtbefestigungen
31. Leuchte (zur Pupillenfunktionsüberprüfung)
32. Fingersensor
33. Oberschale
34. Sensorenpaar Blutfluß (Lichtsender und -empfänger)
35. Führungsschiene
36. Andruckvorrichtung
37. Andruckführung
38. Andruckwelle
39. Andruckverstellung und -Messung
40. Andruckmotor
41. Sensorenpaar Wandspannung (Lichtsender und -empfänger)
42. Elastische Befestigung Schalltrichter
43. Druckaufnehmer
44. Schlägellinearachse mit Rückstellfeder
45. Elektromagnetischer Linearantrieb
46. Achsenführung mit Feder
47. Schlägelplatte

## Patentansprüche

1. Vorrichtung zur Untersuchung und Überwachung von Vitelparametern des menschlichen oder tierischen Körpers, bestehend aus einem Gehäuse, in dem Vorrichtungen (1, 2", 3, 4, 5, 34, 39, 41, 43, 26, 47) zur Aufnahme und Weiterleitung von Signalen des Körpers eingesetzt sind, sowie eine Vorrichtung zur elektronischen Auswertung, Filterung und Speicherung der von diesen Aufnahmevorrichtungen gelieferten Signale und Vorrichtungen zur visuellen Wiedergabe in digitaler oder analoger Form (7, 14, 15, 16) oder zur akustischen Wiedergabe (9) der ausgewerteten Signale, wobei an den Außenseiten des Gehäuses Schnittstelle (10, 11, 20, 22) zum Anschluß externer Vorrichtungen und Geräte angeordnet sind, **dadurch** gekenntzeichnet, daß eine der Vorrichtungen zur Aufnahme von Signalen des Körpers eine Aufnahmeeinheit für akustische Signale ist, bestehend aus einem Schalltrichter (1), einer Membran (2") und einem Mikrophon (3), wobei die Membran (2") an der Unterseite des Gehäuses angeordnet ist, die zum Aufsetzen auf die untersuchten Körperbereich dient, und daß die Vorrichtung ein Perkussionselement als Schallerzeugungsteil (27', 44 bis 47) enthält, das einen Schlägel (27') mit einem elektromagnetischen Antrieb (44 bis 46) zur Schallerzeugung aufweist, der auf eine Anschlagfläche (47) an der Unterseite des Gehäuses anschlägt.

2. Vorrichtung nach Anspruch 1, **dadurch gekennzeichnet, daß** die Vorrichtungen zur visuellen Wiedergabe der ausgewerteten Signale aus Bedien- und Anzeigefeidern mit Dieplay-Digitalfeldern (15), Displayamplitudenbalken (16) und den erforderlichen Bedientasten (13) sowie einem im Bedien- und Anzelgefeld integrierten oder diesem zugeordneten, am Gehäuse der Vorrichtung angesetzten Blidschirm (7) mit Displaykurvenfeldern (14) bestehen, wobei in den Anzeigefeldern mehrere Signale, und zwar gleichzeitig oder ungleichzeitig aufgenommen, dargestellt werden können, wobei die Bedien- und Anzeigefelder (13, 15, 16) und der Bildschirm (7) an der dem Körper abgewandten oberen Seite der Vorrichtung angebracht sind.

3. Vorrichtung nach Anspruch 1 oder Anspruch 2, **dadurch gekennzeichnet, daß** die Vorrichtung zur akustischen Wiedergabe ein im Gehäuse integrierter Lautsprecher (9) mit Verstärker und Alarmmelder ist, der insbesondere an der oberen, vom Körper abgewandten Seite der und angebracht ist.

4. Vorrichtung nach Anspruch 1 oder Anspruch 2, **dadurch gekennzeichnet, daß** eine der Vorrichtungen zur Aufnahme von Signalen das Körpers eine Aufnahmeeinheit für elektrische Signale in Form von Spannungsschwankungen und/oder von impedanzwerten und/oder für Temperaturwerte ist, bestehend aus zwei oder mehreren Meßelektroden (4, 5; ), die an der UnterBeite des Gehäuses angeordnet sind.

5. Vorrichtung nach Anspruch 1, **dadurch gekennzeichnet, daß** die Vorrichtung eine Aufnahmeeinheit mit einem Einlaß zum Einschieben eines Fingerabschnitts hat, wobei der Fingersbschnitt mit seiner einen Seite an einer Außenwand des Moduls und mit der ihr gegenüberliegenden Seite an einer Schale (33) anliegt, die mit einer Andruckvorrichtung (38 bis 40) verbunden und mit dieser in der Führungsschiene (35) waagerecht beweglich geführt ist und wobei die Schale (33) von der Andruckwelle (38) und dem Andruckmotor (40) gegen den eingesetzten Fingerabschnitt gedrückt wird unter Regelung und Messung der Andruckkraft über ein Regelelement (39) an der Andruckwelle (38), daß außerdem an der Schale (33) in Richtung auf den einzusetzenden Fingerabschnitt ein Lichtsender (34) angesetzt ist, der mit dem Lichtempfänger (34') auf der gegenüberliegenden Außerwand des Sensormoduls zusammenwirkt.

6. Vorrichtung nach Anspruch 4, **dadurch gekennzeichnet, daß** die Meßelektroden (4) an der Unterseite des Gehäuses der Vorrichtung geringfügig nach innen zurückgesatzt sind und jeweils einen Kontaktmittelraum (5) aufweisen zur Einfüllung eines kontaktmittels wie z. B. Kontaktpaste.

7. Vorrichtung nach Anspruch 4, **dadurch** gekennzeichne5t, daß die Meßelektroden (4') an der Unterseite des Gehäuses eine leicht gewölbte Oberfläche haben und/oder die Meßelektroden (4'") eine gerade Oberfläche aufweisen.

## Claims

1. Device for examining and monitoring vital parameters of the human or animal body, consisting of a case in which component (1, 2", 3, 4, 5; 34, 39, 41, 43, 26, 47) are employed to record and transfer body signals as well as a component for the electronic evaluation, filtering and saving of the signals provided by these recording components and components for the visual replay in digital or analogue form (7, 14, 15, 16) or for the acoustic replay (9) of the evaluated signals, whereby the exteriors of the case contain interfaces (10, 11, 20, 22) for connecting external devices and units, **characterized in that** one of the components for recording body signals is a retarding unit for acoustic signals consisting of a horn (1), a a membrane (2") and a microphone (3), whereby the membrane (2") is located on the underside of the case which serves to be attached to the parts of the body being examined, and that the component contains a percussion element as a sound generation part (27', 44 to 47), which features a stick (27') with an electromagnetic drive unit (44 to 46) for sound generation that strikes a ringing surface on the underside of the case.

2. Device according to claim 1, **characterized in that** the components for the visual replay of the evaluated signals consist of operating panels and display panels with display digital panels (15), display amplitude bars (16) and the required operating keys (13) as well as a screen (7) with display trend areas (14) that is attached to the case of the device and that is integrated in the operating- and controldisplay panel or assigned to it, whereby several signals that are recorded simultaneously or separately can be displayed on the display panels, and whereby the control panels and display panels (13, 15, 16) and the screen (7) are mounted on the upper side of the device that is faced away from the body.

3. Device according to claim 1 or claim 2, **characterized in that** the component for the acoustic replay is a loudspeaker (9) with an amplifier and alarm indicator that is integrated in the case and particularly mounted on the upper side of the device that is faced away from the body.

4. Device according to claim 1 or claim 2, **characterized in that** one of the components for recording body signals is a recording unit for electrical signals in the form of voltage fluctuations and/or of impedance values and/or for temperature values, consisting of two or more measuring electrodes (4, 5) that are located on the underside of the case.

5. Device according to claim 1, **characterized in that** the device has a taking unit with an entrance for inserting a part of a finger, whereby the part of the finger has one of its sides touching an external wall of the module and the opposite side touching a pan (33), which is connected to a pressing device (36 to 40) and which is guided horizontally movable with this in the track (35) and where the pan (33) is pressed against the inserted part of the finger by the pressure shaft (38) and the pressure motor (40) while regulating and measuring the pressing force via a control element (39) on the pressure shaft (38), and that moreover a light emitter (34) is attached to the pan in the direction of the part of the finger to be inserted, which works together with the light-receiver (34') on the opposite external wall of the sensor module.

6. Device according to claim 4, **characterized in that** the measuring electrodes (4) are relocated slightly inward on the underside of the device case and each feature a contact means area (5) for filling in a contact means such as contact paste, for example.

7. Device according to claim 4, **characterized in that** the measuring electrodes (4') on the underside of the case have a slightly curved surface and/or the measuring electrodes (4"') have an even surface.

## Revendications

1. Dispositif d'examen et de contrôle des paramètres vitaux du corps humain ou animal, composé d'un coffret, dans lequel sont placés des dispositifs (1, 2", 3, 4, 5, 34, 39, 41, 43, 26, 47) pour le captage et la tansmission de signaux du corps, ainsi qu'un dispositif d'analyse électronique, filtration et enregistre-ment des signaux émis par ces dispositifs de captage et des dispositifs de reproduction visuelle sous forme numérique ou analogue (7, 14, 15, 16) ou de reproduction acoustique (9) des signaux analysés, des points de liaison (10, 11, 20, 22) étant disposés sur les côtés extérieurs du coffret pour raccorder des dispositifs et appareils externes, **caractérisé en ce que** l'un des dispositifs de captage des signaux du corps est une unité de captage de signaux acoustiques, composé d'un cornet (1), d'une membrane (2") et d'un microphone (3), la membrane (2") étant placée sur le côté inférieur du coffret qui sert à être posé sur les parties examinées du corps, et **en ce que** le dispositif comporte un élément de percussion en tant qu'élément de production de sons (27', 44 à 47), ayant un battoir (27') doté d'une commande électromagnétique (44 à 46) pour la production de sons, frappant sur une surface de butée (47) située sur le côté inférieur du coffret.

2. Dispositif selon la revendication 1, **caractérisé en ce que** les dispositifs de reproduction visuelle des signaux analysés se composent de zones de commande et d'affichage avec des panneaux numériques d'affichage (15), des barres d'amplitude digitale d'affichage (16) et les touches de commande (13) requises ainsi que d'un écran (7) avec courbes d'affichage (14), cet écran étant ajouté au coffret du dispositif et intégré dans la zone de commande et d'affichage ou en faisant partie; plusieurs signaux, captés de manière synchrone ou asynchrone pouvant être représentés dans les zones d'affichage, les zones de commande et d'affichage (13, 15, 16) et l'écran (7) étant placés sur le côté supérieur et opposé au corps du dispositif.

3. Dispositif selon la revendication 1 ou la revendication 2, **caractérisé en ce que** le dispositif de reproduction acoustique est un haut-parleur (9) intégré dans le coffret avec amplificateur et avertisseur d'alarme, notamment placé sur le côté supérieur du dispositif et opposé au corps.

4. Dispositif selon la revendication 1 ou la revendication 2, **caractérisé en ce que** l'un des dispositifs de captage des signaux du corps est une unité de captage de signaux électriques sous forme de variations de tension et/ou d'indices d'impédance et/ou de température, composée de deux ou plusieurs électrodes de mesurage (4, 5) disposées sur le côté inférieur du coffret.

5. Dispositif selon la revendication 1, **caractérisé en ce que** le dispositif possède une unité de captage dotée d'un orifice pour introduire un bout de doigt, le bout de doigt s'appuyant d'un côté sur une paroi extérieure du module et du côté en face sur une gaine (33) refilée à un dispositif de compression (38 à 40) et conduite avec lui horizontalement librement dans le coulisseau (35), la gaine (33) étant poussée par l'arbre de pression (38) et le moteur de pression (40) contre le bout de doigt inséré en réglant et mesurant la force de pression au moyen d'un élément de réglage (39) sur l'arbre de pression (38), **en ce qu'**en plus un émetteur lumineux (34) est placé sur la gaine (33) dans le sens du bout de doigt inséré et agit avec le récepteur lumineux (34') sur la paroi extérieure opposée du module sensitif.

6. Dispositif selon la revendication 4, **caractérisé en ce que** les éléctrodes de mesurage (4) sur le côté inférieur du coffret du dispositif sont légèrement déplacées vers l'intérieur et présentent respectivement un compartiment pour produit de contact (5) pour introduire un produit de contact, tel que par ex. une pâte de contact.

7. Dispositif selon la revendication 4, **caractérisé en ce que** les éléctrodes de mesurage (4') sur le côté inférieur de coffret ont une surface légèrement bombée et/ou que les électrodes de mesurage (4"') présentent une surface Plate.
